# EUROPÄISCHE PATENTSCHRIFT

(11) **EP 0 752 412 B1**
(45) Veröffentlichungstag und Bekanntmachung des Hinweises auf die Patenterteilung: **23.12.1998**
(21) Anmeldenummer: 95110244.1
(22) Anmeldetag: 30.06.1995
(51) Int. Cl.: C07C 215/00, C07C 213/04

(54) **Verfahren zur Herstellung von farbstabilem Dialkylaminoethanol**
Process for the preparation of colour stable dialkylaminoethanols
Procédé pour le préparation des dialkylaminoéthanols avec stabilité de la couleur

(43) Veröffentlichungstag der Anmeldung: 08.01.1997
(73) Patentinhaber: BASF Aktiengesellschaft, 67063 Ludwigshafen (DE)
(72) Erfinder: Melder, Johann-Peter, Dr., D-68165 Mannheim (DE); Ruider, Günther, Dr., D-67157 Wachenheim (DE); Witzel, Tom, Dr., D-67069 Ludwigshafen (DE); Ross, Karl-Heinz, Dr., D-67269 Grünstadt (DE); Boettger, Günter, Dr., D-67098 Bad Dürkheim (DE)

(56) Entgegenhaltungen:
- EP-A- 0 070 978
- DD-A- 203 534
- FR-A- 2 251 545
- FR-A- 2 387 212
- US-A- 3 131 132

## Beschreibung

Die vorliegende Erfindung betrifft ein Verfahren zur Herstellung von farbstabilem Dialkylaminoethanol aus Dialkylamin und Ethylenoxid durch Umsetzung in Gegenwart von 2,5 bis 25 Gew.-% Wasser bei erhöhten Temperaturen und die Abtrennung des Dialkylamins durch Destillation bei einer Sumpftemperatur von bis zu 90°C.

Alkylaminoethanole sind wichtige Zwischenprodukte für die chemische und pharmazeutische Industrie. Dimethylaminoethanol findet z.B. in Form von Salzen, Seifen, Ether und Ester als Emulgator und oberflächenaktive Substanz sowie als Katalysator in der Polyurethanchemie Anwendung auf den verschiedensten Gebieten. In der pharmazeutischen Industrie wird es zur Synthese von Wirkstoffen (Tranquilizer, Antihistaminika und Analgetika) benutzt. Verfärbungen in Dimethylaminoethanol sind bei den meisten Anwendungen unerwünscht.

Die Anlagerung von Amine an Ethylenoxid wird, wie beim Ammoniak durch Zusatz von Wasser erheblich beschleunigt. So ist zur Umsetzung von Ethylenoxid mit Dimethylamin ohne Wasser ein mehrstündiges Erhitzen auf 150°C notwendig, dagegen erfolgt die Vereinigung zu Dimethylaminoethanol bereits in der Kälte, wenn eine wäßrige Dimethylamin-Lösung verwendet wird. Eine dem Wasser ähnliche Wirkung haben auch Alkohole wie Methanol oder Ethanol. (Houben Weyl, Methoden der organischen Chemie, Band 11/1, 1957, 311 bis 350).

Es ist bekannt, daß Alkanolamine durch Ethoxylierung der Hydroxyl-gruppe zu höherethoxylierten Produkten weiterreagieren. Diese Folgereaktion soll durch Wasser begünstigt werden, sie kann jedoch durch Einsatz von überschüssigem Amin (1,1 : 1 bis 4 : 1) weitgehend zurückgedrängt werden (DE-A-23 57 076; DD-A-203 534, US-A-2 337 004, US-A-2 373 199).

Es ist weiterhin bekannt, daß tertiäre Amine, wie z.B. Dimethylaminoethanol, sowohl in Abwesenheit als auch in Gegenwart von Wasser unterhalb von 80°C mit Oxiranen zu thermisch labilen quartären Ammoniumverbindungen reagieren, die sich oberhalb von 90°C mehr oder weniger rasch zersetzen. (E. Tobler et al. Helv. Chim Acta 52, 1969, Seite 408 bis 418).

Aus der EP-A-70 978 ist die kontinuierliche Umsetzung von überschüssigem Dimethylamin (2,2 eq) mit Ethylenoxid in Gegenwart von Wasser (0,2 bis 0,5 eq) bei 150°C und anschließende destillative Aufarbeitung unter Zusatz definierter Mengen Natriumborhydrid bekannt.

Aus der US-A-3 131 132 ist die diskontinuierliche Umsetzung von überschüssigem Dimethylamin (1 bis 2 eq) mit Ethylenoxid in Gegenwart von Wasser (3 bis 15 eq) bei 50 bis 100°C und anschließende destillative Aufarbeitung (190 mbar) nach Einstellung des pH-Wertes auf 11,5 bekannt.

Aus der JP-A-01/160 947 ist die wasserkatalysierte Synthese von Dimethylethanolamin und die anschließende Aufarbeitung durch destillative Hochsiederabtrennung (100 mbar), Hydrierung des Destillates an Ru/C und Reindestillation bei 100 mbar bekannt.

Aus der DD-A-203 534 ist die Umsetzung von überschüssigem Dimethylamin (1,1 bis 3,5 : 1) mit Ethylenoxid in Gegenwart katalytischer Mengen Wasser (0,02 bis 0,15 eq) bei sehr milden Reaktionsbedingungen (50 bis 90°C) und anschließende destillative Aufarbeitung mit max. Blasentemperatur von 90°C bekannt.

Absenkung der Reaktionstemperatur unter 90°C führt nach Helv. Chim Acta 52, 1969, 408 bis 418 zur Bildung quartärer Basen, was zu Ausbeuteverlusten führt.

Aus der US-A-3 567 779 ist die Inhibierung der Verfärbung durch Zusatz von Mono- oder Diethanolamin zu Dimethylethanolamin bekannt.

Die voranstehenden Lösung zum Erhalt eines farbstabilen Dialkylethanolamins haben die Nachteile des Zusatzes eines fremden Stabilisators, der die Dialkylethanolamine verunreinigt oder des Zusatzes eines Reinigungsstoffes (Reduktionsmittel oder Säure), dessen Abtrennung nur unter größtem Aufwand quantitativ durchgeführt werden kann.

Der vorliegenden Erfindung lag daher die Aufgabe zugrunde, den zuvor genannten Nachteilen abzuhelfen.

Demgemäß wurde ein neues und verbessertes Verfahren zur Herstellung von farbstabilem Dialkylaminoethanol der allgemeinen Formel I 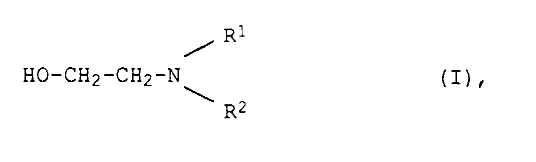 in der R¹ und R² unabhängig voneinander C₁- bis C₂₀-Alkyl bedeuten, aus Dialkylamin der allgemeinen Formel II 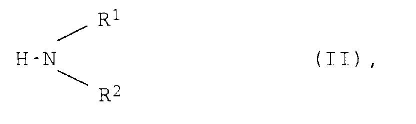 in der R¹ und R² die obengenannten Bedeutungen haben, und Ethylenoxid, dadurch gekennzeichnet, daß man die Umsetzung in Gegenwart von 2,5 bis 50 Gew.-% Wasser, bezogen auf das Reaktionsgemisch, bei Temperaturen von 100 bis 140°C und die Abtrennung eines Gemisches von Wasser und Hochsiedern durch Destillation bei einer Sumpftemperatur von 40 bis 90°C durchführt und man der Reaktionsmischung vor der Destillation nicht mehr als 1 mol Alkalimetall Borhydrid pro 1650 mol des bei der Reaktion eingesetzten Ethylenoxids beifügt.

Das erfindungsgemäße Verfahren kann wie folgt durchgeführt werden:

Die Umsetzung der Dialkylamine II, in der R¹ und R² unabhängig voneinander C₁- bis C₂₀-Alkyl, bevorzugt C₁- bis C₈-Alkyl, besonders bevorzugt C₁- bis C₄-Alkyl, insbesondere Methyl oder Ethyl, ganz besonders Methyl bedeuten, mit Ethylenoxid kann kontinuierlich oder diskontinuierlich bei Temperaturen von 100 bis 140°C, und Drücken von 1,5 bis 100 bar, bevorzugt 5 bis 70 bar, besonders bevorzugt 10 bis 40 bar in Gegenwart von 2,5 bis 50 Gew-%, bevorzugt 5 bis 35 Gew-%, besonders bevorzugt 8 bis 25 Gew.-% Wasser bezogen auf das Reaktionsgemisch durchgeführt werden.

Das Molverhältnis von Dialkylamin II zum Ethylenoxid beträgt in der Regel 1 : 1 bis 50 : 1, bevorzugt 1,1 : 1 bis 20 : 1, besonders bevorzugt 1,2 : 1 bis 10 : 1.

Überschüssiges Dialkylamin wird destillativ abgetrennt und bevorzugt in das Verfahren zurückgeführt. Die destillative Abtrennung von Wasser und hochsiedenden Nebenprodukten (quartäre Basen, Dialkylaminodiglykol) wird bei Sumpftemperaturen von 40 bis 90°C, bevorzugt 55 bis 90°C, besonders bevorzugt 60 bis 85°C und unter einem Druck von 5 bis 150 mbar, bevorzugt 25 bis 150 mbar, besonders bevorzugt 50 bis 150 mbar durchgeführt.

Die bei dem erfindungsgemäßen Verfahren erhältlichen Dialkylethanolamine haben in der Regel eine Reinheit von 99 bis 99,999 %, bevorzugt 99,5 bis 99,99 %, besonders bevorzugt 99,8 bis 99,9 % und sind bei Lagerung unter Inertatmosphäre (Stickstoff, Argon) über Monate verfärbungsstabil.

### Beispiele

### Vergleichsbeispiel 1

In einem 300 ml Rührreaktor aus V2A-Stahl mit Druck- und Temperaturanzeige werden 99 g (2,2 mol) Dimethylamin und 1,1 g (0,06 mol) Wasser vorgelegt und auf 65°C vorgewärmt. Unter Rühren werden zunächst 5 g Ethylenoxid in die Flüssigphase dosiert, wobei nach wenigen Minuten durch Temperaturerhöhung das Anspringen der Reaktion angezeigt wird. Nun wird der Rest von insgesamt 48 g (1,1 mol) Ethylenoxid innerhalb von 60 min zudosiert. Durch externe Kühlung wird die Reaktionstemperatur zwischen 74 und 80°C gehalten. Nach Dosierende wird noch 5 Minuten nachgerührt, die Reaktionsmischung auf 50°C abgekühlt, vorsichtig entspannt und überschüssiges DMA bei 50°C durch N₂-Strippung entfernt. Man erhält 93,0 g ethylenoxidfreies Reaktionsprodukt mit folgender Zusammensetzung:

| | |
|---|---|
| Dimethylethanolamin | 80,52 % |
| Dimethylaminodiglykol | 5,28 % |
| Quartäre Basen | 10,00 % |
| Sonstige Produkte | 3,10 % |
| Wasser | 1,10 % |

Destillation des Rohaustrages über eine 35 cm Füllkörperkolonne bei einem Vakuum von 50 - 100 mbar liefert DMEA mit einer Farbzahl von 5 APHA. Die Beurteilung der Farbstabilität erfolgt über folgenden Test: Bestimmung der Farbzahl nach Erhitzen von Rein-DMEA für 6 h bei 60°C unter Stickstoff ----> 5 APHA.

### Vergleichsbeispiel 2

In einem 300 ml Rührreaktor aus V2A-Stahl mit Druck- und Temperaturanzeige werden 99 g (2,2 mol) Dimethylamin und 1,1 g (0,06 mol) Wasser vorgelegt und auf 65°C vorgewärmt. Unter Rühren werden zunächst 5 g Ethylenoxid in die Flüssigphase dosiert, wobei nach wenigen Minuten durch Temperaturerhöhung das Anspringen der Reaktion angezeigt wird. Nun wird der Rest von insgesamt 48 g (1,1 mol) Ethylenoxid innerhalb von 120 min zudosiert. Durch externe Kühlung wird die Reaktionstemperatur zwischen 74 und 80°C gehalten. Nach Dosierende wird noch 5 Minuten nachgerührt, die Reaktionsmischung auf 50°C abgekühlt, vorsichtig entspannt und überschüssiges DMA bei 50°C durch N₂-Strippung entfernt. Man erhält 95,0 g ethylenoxidfreies Reaktionsprodukt mit folgender Zusammensetzung:

| | |
|---|---|
| Dimethylethanolamin | 87,17 % |
| Dimethylaminodiglykol | 3,28 % |
| Quartäre Basen | 5,63 % |
| Sonstige Produkte | 2,82 % |
| Wasser | 1,10 % |

Destillation des Rohaustrages über eine 35 cm Füllkörperkolonne bei einem Vakuum von 50 bis 100 mbar liefert DMEA mit einer Farbzahl von 5 APHA. Farbzahl nach 6h/60°C: 5 APHA.

### Beispiel 1

In einem 500 ml Rohrreaktor mit Druck- und Temperaturanzeige wird ein auf 75°C vorgewärmtes Gemisch aus Dimethylamin (3350 g/h; 74,5 mol/h) und Wasser (840 g/h; 46,7 mol/h) kontinuierlich mit 600 g/h (13,6 mol/h) Ethylenoxid umgesetzt. Durch externe Kühlung führt man die Reaktionswärme dabei so ab, daß die Reaktionstemperatur 110°C nicht überschreitet. Nach destillativer Abtrennung und Rückführung von überschüssigem Dimethylamin (3 bis 4 bar/T_{Sumpf} 130 bis 140°C) und Wasser (Azeotrop mit Dimethylethanolamin; 600 mbar/T_{Sumpf} 100 bis 105°C) erhält man 1275 g/h ethylenoxidfreies Reaktionsprodukt mit folgender Zusammensetzung:

| | |
|---|---|
| Dimethylethanolamin | 82,64 % |
| Dimethylaminodiglykol | 2,40 % |
| Quartäre Basen | 3,67 % |
| Sonstige Produkte | 3,55 % |
| Restwasser | 7,74 % |

Destillative Aufarbeitung (Abtrennung von Hochsiedern und Restwasser) bei 50 mbar (T_{Sumpf} 65 bis 75°C) erhält man Dimethylethanolamin in 99,8 bis 99,9 %iger Reinheit mit einer Farbzahl von 5 APHA. Farbzahl nach 6h/60°C: 5 APHA, Farbzahl nach 3 Monaten bei Raumtemperatur unter Stickstoff: 10 APHA.

### Beispiel 2

In einem 300 ml Rührreaktor aus V2A-Stahl mit Druck- und Temperaturanzeige werden 90 g (2 mol) Dimethylamin und 22,5 (2,5 mol) Wasser vorgelegt und auf 120°C vorgewärmt. Unter Rühren werden dann innerhalb von 10 min 22 g (0,5 mol) Ethylenoxid zudosiert. Die Temperatur steigt dabei von 120 auf 140°C. Nach Dosierende wird 5 Minuten bei 140°C nachgerührt, die Reaktionsmischung auf 50°C abgekühlt, entspannt und überschüssiges Dimethylamin und ein Teil des Wassers durch N₂-Strippung entfernt. Man erhält 50,8 g ethylenoxidfreies Reaktionsprodukt mit folgender Zusammensetzung:

| | |
|---|---|
| Dimethylethanolamin | 84,45 Gew.-% |
| Dimethylaminodiglykol | 1,00 Gew.-% |
| Quartäre Basen | < 0,10 Gew.-% |
| Sonstige Produkte | 1,55 Gew.-% |
| Restwasser | 13,00 Gew.-% |

Destillation des Rohaustrages über eine 35 cm Füllkörperkolonne bei einem Vakuum von 50 mbar liefert DMEA mit einer Farbzahl von 5 APHA. Farbzahl nach 6h/60°C : 5 APHA.

## Patentansprüche

1. Verfahren zur Herstellung von farbstabilem Dialkylaminoethanol der allgemeinen Formel I 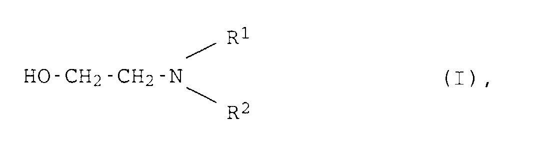 in der R¹ und R² unabhängig voneinander C₁- bis C₂₀-Alkyl bedeuten, aus Dialkylamin der allgemeinen Formel II 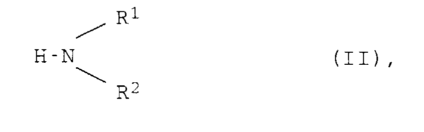 in der R¹ und R² die obengenannten Bedeutungen haben, und Ethylenoxid, dadurch gekennzeichnet, daß man die Umsetzung in Gegenwart von 2,5 bis 50 Gew.-% Wasser, bezogen auf das Reaktionsgemisch, bei Temperaturen von 100 bis 140°C und die Abtrennung eines Gemisches von Wasser und Hochsiedern durch Destillation bei einer Sumpftemperatur von 40 bis 90°C durchführt und man der Reaktionsmischung vor der Destillation nicht mehr als 1 mol Alkalimetall Borhydrid pro 1650 mol des bei der Reaktion eingesetzten Ethylenoxids beifügt.

2. Verfahren zur Herstellung von farbstabilem Dialkylaminoethanol I nach Anspruch 1, dadurch gekennzeichnet, daß R¹ und R² C₁- bis C₈-Alkyl bedeuten.

3. Verfahren zur Herstellung von farbstabilem Dialkylaminoethanol I nach Anspruch 1, dadurch gekennzeichnet, daß R¹ und R² C₁- bis C₄-Alkyl bedeuten.

4. Verfahren zur Herstellung von farbstabilem Dialkylaminoethanol I nach Anspruch 1, dadurch gekennzeichnet, daß R¹ und R² Methyl oder Ethyl bedeuten.

5. Verfahren zur Herstellung von farbstabilem Dialkylaminoethanol I nach Anspruch 1, dadurch gekennzeichnet, daß R¹ und R² Methyl bedeutet.

6. Verfahren zur Herstellung von farbstabilem Dialkylaminoethanol I nach Anspruch 1, dadurch gekennzeichnet, daß man die Abtrennung von Wasser und Hochsiedern durch Destillation bei Drücken von 5 bis 150 mbar und einer Sumpftemperatur von 40 bis 90°C durchführt.

7. Verfahren zur Herstellung von farbstabilem Dialkylaminoethanol I nach Anspruch 1, dadurch gekennzeichnet, daß man die Abtrennung von Wasser und Hochsiedern durch Destillation bei Drücken von 25 bis 150 mbar und einer Sumpftemperatur von 40 bis 90°C durchführt.

8. Verfahren zur Herstellung von farbstabilem Dialkylaminoethanol I nach Anspruch 1, dadurch gekennzeichnet, daß man die Abtrennung des Dialkylamins II durch Destillation bei einer Sumpftemperatur von 55 bis 140°C durchführt.

9. Verfahren zur Herstellung von farbstabilem Dialkylaminoethanol I nach Anspruch 1, dadurch gekennzeichnet, daß man die Abtrennung von Wasser und Hochsiedern durch Destillation bei einer Sumpftemperatur von 60 bis 85°C durchführt.

## Claims

1. A process for the preparation of stable-colored dialkylaminoethanol of the general formula I 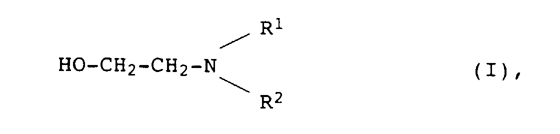 in which R¹ and R² independently are C₁-C₂₀-alkyl, from dialkylamine of the general formula II 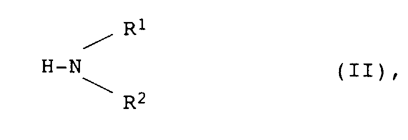 in which R¹ and R² are as defined above, and ethylene oxide, which comprises carrying out the reaction in the presence of from 2.5 to 50 % by weight of water, based on the reaction mixture, at from 100 to 140°C, separating off a mixture of water and high-boiling constituents by distillation at from 40 to 90°C at the column bottom and, prior to the distillation, adding to the reaction mixture not more than 1 mol of alkali metal boron hydride per 1650 mol of the ethylene oxide used in the reaction.

2. The process as claimed in claim 1, wherein R¹ and R² are C₁-C₈-alkyl.

3. The process as claimed in claim 1, wherein R¹ and R² are C₁-C₄-alkyl.

4. The process as claimed in claim 1, wherein R¹ and R² are methyl or ethyl.

5. The process as claimed in claim 1, wherein R¹ and R² are methyl.

6. The process as claimed in claim 1, wherein water and high-boiling constituents are separated off by distillation at from 5 to 150 mbar and at from 40 to 90°C at the column bottom.

7. The process as claimed in claim 1, wherein water and high-boiling constituents are separated off by distillation at from 25 to 150 mbar and at from 40 to 90°C at the column bottom.

8. The process as claimed in claim 1, wherein the dialkylamine II is separated off by distillation at from 55 to 90°C at the column bottom.

9. The process as claimed in claim 1, wherein water and high-boiling constituents are separated off by distillation at from 60 to 85°C at the column bottom.

## Revendications

1. Procédé de préparation de dialkylaminoéthanol à couleur stable de formule générale I 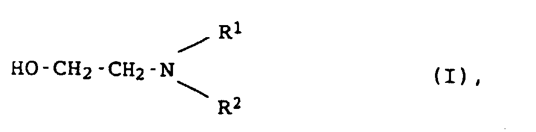 dans laquelle R¹ et R² représentent indépendamment l'un de l'autre un groupement alkyle en C₁-C₂₀, à partir de dialkylamines de formule générale II 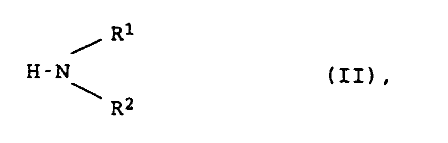 dans laquelle R¹ et R² prennent la signification susmentionnée, et d'oxyde d'éthylène, caractérisé en ce que l'on mène la réaction en présence de 2,5-50% en poids d'eau, par rapport au mélange réactionnel, à des températures de 100-140°C et l'on mène la séparation d'un mélange d'eau et de composés à hauts points d'ébullition par distillation à une température de fond de 40-90°C et on n'ajoute au mélange réactionnel, avant la distillation, pas plus d'une mole de borhydrure de métal alcalin pour 1650 moles de l'oxyde d'éthylène utilisé lors de la réaction.

2. Procédé de préparation de dialkylaminoéthanol à couleur stable de formule générale I selon la revendication 1, caractérisé en ce que R¹ et R² représentent des groupements alkyle en C₁-C₈.

3. Procédé de préparation de dialkylaminoéthanol à couleur stable de formule générale I selon la revendication 1, caractérisé en ce que R¹ et R² représentent des groupements alkyle en C₁-C₄.

4. Procédé de préparation de dialkylaminoéthanol à couleur stable de formule générale I selon la revendication 1, caractérisé en ce que R¹ et R² représentent des groupements méthyle ou éthyle.

5. Procédé de préparation de dialkylaminoéthanol à couleur stable de formule générale I selon la revendication 1, caractérisé en ce que R¹ et R² représentent des groupements méthyle.

6. Procédé de préparation de dialkylaminoéthanol à couleur stable de formule générale I selon la revendication 1, caractérisé en ce que l'on mène la séparation de l'eau et des composés à hauts points d'ébullition par distillation sous des pressions de 5-150 mbar et à une température de fond de 40-90°C.

7. Procédé de préparation de dialkylaminoéthanol à couleur stable de formule générale I selon la revendication 1, caractérisé en ce que l'on mène la séparation de l'eau et des composés à hauts points d'ébullition par distillation sous des pressions de 25-150 mbar et à une température de fond de 40-90°C.

8. Procédé de préparation de dialkylaminoéthanol à couleur stable de formule générale I selon la revendication 1, caractérisé en ce que l'on mène la séparation de la dialkylamine II par distillation à une température de fond de 55-90°C.

9. Procédé de préparation de dialkylaminoéthanol à couleur stable de formule générale I selon la revendication 1, caractérisé en ce que l'on mène la séparation de l'eau et des composés à hauts points d'ébullition par distillation à une température de fond de 60-85°C.
